# EUROPEAN PATENT APPLICATION

(11) **EP 2 153 783 A1**
(43) Date of publication of application: **17.02.2010**
(21) Application number: 07789605.8
(22) Date of filing: 05.06.2007
(51) Int. Cl.: A61B 17/32

(54) **MEDICAL DEVICE FOR EXPLORING AND OPERATING ON THE ABDOMINAL CAVITY AND THE PELVIC CAVITY**

(71) Applicant: Cárdenas, Marcela, Bogota D.C. (CO); Arango, Rafael, Bogota D.C. (CO); Gonzalez, Jaime, Cartegena (CO)
(72) Inventor: Cárdenas, Marcela, Bogota D.C. (CO); Arango, Rafael, Bogota D.C. (CO); Gonzalez, Jaime, Cartegena (CO)
(74) Representative: Martin Santos, Victoria Sofia
(86) International application number: PCT/IB2007/002240
(87) International publication number: WO 2008/149173

(57) **Abstract**

The MEDICAL DEVICE's main aim it to diagnose with high precision whether a patient suffers an inflammation of the appendix, appendicitis, or any other abdominal pathology before taking the decision of surgical intervention. It can likewise be used for surgical intervention. Both applications have several advantages like the conditions or the environment in which the device can be used, since it does not require operating rooms, highly specialized staff and general anesthesia. Its use as a therapeutic instrument takes place in an operating room, with general anesthesia, but it does not require highly specialized staff. The MEDICAL DEVICE is a greatly simpler and more economic instrument than known medical instruments; it can be easily used after a short training. This device has a trocar with a hollow tip in order to allow the passage of a miniature camera. The trocar has been modified and has an innovative design; it has a source of light and a miniature camera on its tip, which transmits images to a LCD screen. It has two or more lateral working channels so that surgical material can be introduced in case the removal of the appendix or other similarly sized structures must take place. The camera is connected to a cable, which at its turn is connected to a LCD screen, giving the surgeon the impression he or she is looking into the patient's abdominal cavity through a window.

During the diagnosis stage, the device will improve the conventional peritoneal pumping which still takes place in many ERs.

The MEDICAL DEVICE will furthermore minimize the number of exams required for the diagnosis, the time required to adopt a therapeutic strategy, the pain and the patient's discomfort during the diagnosis process, the number of wrong diagnosis, the cost of the diagnosis, both in physical resources and in staff, the documentation related to the process and the time patients remain in observation in order to be diagnosed. It will likewise maximize the intervention of specialists during the diagnosis process and the "visibility" of the abdominal cavity obtained through diagnostic exams.

## Description

### 1.FIELD OF THE INVENTION

This invention is related to a medical device. This device's main finality it to explore the abdominal cavity and the pelvic cavity, in order to diagnose, with high precision, the inflammation or disease of internal organs, in order to confirm or discard a surgical intervention. The instrument allows the view of abdominal and pelvic organs, this allows diagnosing various pathologies which may be detected though direct viewing, similarly to a traditional laparotomy. Likewise, this instrument can also be used to extract the appendix or other similarly sized structures (tubes, ovaries, cysts, samples of biopsies)

### 2.BACKGROUND OF THE INVENTION

Traditionally, diagnostic explorations of abdominal pain and consequent surgical interventions are taken out through laparotomy or laparoscopy. These are recommended in case of a non specific abdominal pain, once other non invasive exams are used up.

Laparotomy is defined as the incision realized on the abdominal wall so that the surgeon can observe and palpate the organs of the abdominal area. It has disadvantages such as the big incision on the abdominal wall, that is, it is an invasive and bloody intervention, with all of the consequences this implies: higher possibility of infections, slower recovery, mayor postoperative pain, mayor risk of complications (hernias, intestinal obstruction). In order to realize this procedure all measures necessary in a surgical room must be taken: a sterile surgical environment, surgical equipment, surgical nurses, specialized laparoscopic surgeons, anesthesiologists and an anesthetic machine.

Laparoscopy is taken out with at least three small incisions on the abdominal wall. In order to be taken out several requirements are necessary: a sterile surgical environment, laparoscopic surgical equipments (a monitor, an insufflator, a camera, an image processor, a source of light), a tray of laparoscopic instruments, surgical nurses, specialized laparoscopic surgeons, anesthesiologists and an anesthetic machine.

This new instrument has several advantages in comparison to the above described methods. On the one hand, this procedure allows the direct viewing of the abdominal and pelvic cavity in a much less invasive way, since it is taken out though a small incision under the bellybutton. If compared with traditional laparotomy, the probabilities of infection decrease, recovery is quicker (less days of hospitalization, the procedure could even be outpatient, less days of inability), less scars, less pain after the procedure. Likewise, this procedure can be used as a diagnostic instrument in ERS - which are clean but not sterile - with local anesthesia, relaxation and tranquilization of the patient. Medical staff taking out the procedure will not need the demanding training of a laparoscopic surgeon. The use of this MEDICAL DEVICE does not require laparoscopic equipment, like the insufflator which creates a virtual space in the abdominal cavity in order to explore it. This space will be created by the MEDICAL DEVICE using arms which open in the cavity and that create the space through mechanic traction. This characteristic offers several medical benefits, since gases are an additional risk which is taken into account before a laparoscopic procedure. With MEDICAL DEVICE this risk does not exist. Its use will diminish costs of diagnostic laparoscopy and will improve the availability of these equipments, since, because of their cost, laparoscopy machines are never in ERs. MEDICAL DEVICE is a simple, portable, light, manually operated and sensibly more economic machine.

MEDICAL DEVICE is an innovation in the laparoscopic field, unlike laparoscopy, however, it is taken out with only one incision. Traditional laparoscopy requires at least three. This means that MEDICAL DEVICE has a less invasive procedure. In addition, the incision in order to introduce MEDICAL DEVICE is made in the umbilical area, which makes the incision practically invisible, once healed. MEDICAL DEVICE has a handle which is connected to the retractor on its tip, allowing the creation of a space in the abdominal cavity without use of gas.

In traditional surgeries, the surgeon must look at a screen which is generally above the patient, while the surgeon's hands work. The surgeon does not see his hands work and does not see the patient's abdomen; the axis eye-hand-objective is broken. This situation complicates the learning of this technique, which requires a long and hard training in order to be mastered. MEDICAL DEVICE eases this apprenticeship, since the screen which allows the interior of the abdomen to be viewed is placed on top of the abdomen, like a window that allows the surgeon to see what occurs naturally and to see his hands moving. This characteristic will allow a major mastership of the technique in less time.

### 3.DESCRIPTION

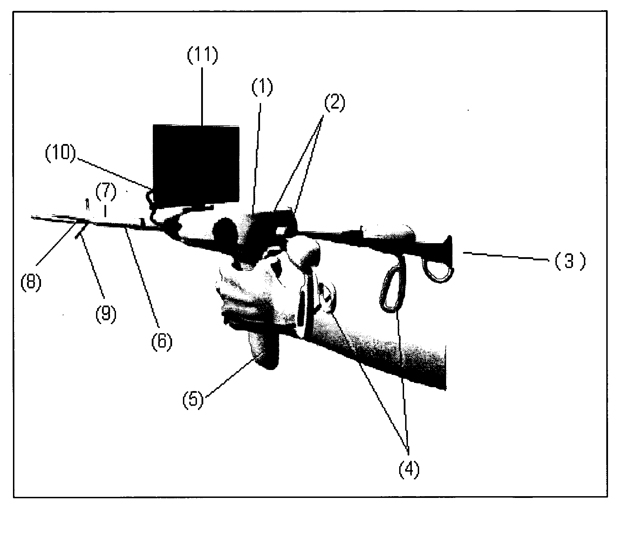

MEDICAL DEVICE is composed by a trocar, which is a surgical instrument passed through the body, used to allow easy exchange of endoscopic instruments during endoscopic surgery, as its main body (1) with a hollow tip (2) with a light source (7), which allows the passage of a miniature camera until the tip (8). This camera transmits the images to a LCD screen (11), as shown in figure 1, which is connected to the main body through a support, which allows this screen to be moved and even removed. The main body (1) of this MEDICAL DEVICE has two lateral working channels (2) in addition to the central one, and included in the main body (1). These are thought of in order to introduce necessary surgical material (4) in case of therapeutic use, so that no additional incisions have to be made. This is why these two lateral channels (2) come out to a common hole (12), through which the medical intervention is taken out and to which both lateral channels (2) and the central one (3) come out to. The miniature camera (8) and the source of light (7) go through the lateral channels (2), which is where surgical instruments are inserted in order to intervene on the patient, and through the central one (3), so that the surgeon can see. At the tip, where the common exit hole is located (12), this MEDICAL DEVICE has two arms (9) which open and close in order to separate tissues and allow intervention on the patient. The camera (8) that goes through the central channel (3) is connected to a cable, which is, in turn, connected to a LCD screen (11), giving the surgeon the impression of looking inside the patient's abdominal cavity as through a mirror, and hence keeping the eye-hand-objective axis, so that who uses the MEDICAL DEVICE does not have to look elsewhere as occurs with other systems. The MEDICAL DEVICE is held by an ergonomic handle (5) in order to ease its use, so that it becomes a versatile and portable instrument. Despite the existence of similar components on the market, such as the trocar, the miniature camera, the LCD screen, the way these are assembled, the modifications and the use give to the device, gives it an innovative quality in comparison with the previous technique, especially because it has three independent channels that come out to one common hole.

### 1. For diagnostic use

During diagnosis, the doctor makes a small incision from 1.5 to 2 cm. under the bellybutton and inserts the MEDICAL DEVICE in the resuscitation room, a non sterile environment. The patient must be sedated, relaxed and with local anesthesia. Once it appears on the screen that the abdominal cavity has been entered, the doctor will turn on a control of the MEDICAL DEVICE in order to open the arms on its tip. This will allow the creation of a space similar to the pneumoperitoneum. After revision of the cavity, if there are doubts on the diagnose or if specific conditions have to be studied, a saline solution is injected and then recovered in order to be studied. In this way, apart from the recovery of the saline solution, the abdominal cavity can be explored directly and in ER in order to increase the precision of the examination.

### 2. For therapeutic use

In order to remove the appendix or other similarly sized structures (tubes, ovaries, cysts, samples of biopsies), the surgeon must insert a suture with a previously prepared knot through one of the MEDICAL DEVICE's working channels, wrap the structure and then tie the knot. Then, the sugeon repeats the maneuver and inserts endoscopic scissors through the working channel and cuts between the two knots. Afterwards, the surgeon will remove the structure through the very incision created in order to insert the MEDICAL DEVICE in the bellybutton. The maximum size of this incision will be of 2 centimeters, it will eventually become invisible thanks to its location and size.

Unlike the laparoscopy, with the MEDICAL DEVICE three small incisions are not required. Apart from the cosmetic benefits this tool offers, the patient can return home on the same day, and even work the following day. The patient's pain will decrease, reducing the need of painkillers.

### 4.BRIEF DESCRIPTION OF THE DRAWING

FIGURE 1: Tridimensional figure where the MEDICAL DEVICE and its components can be appreciated which comprises the following.

- 1: Main Body: Trocar
- 2: Lateral working channels
- 3: Central channel
- 4: Surgical instruments
- 5: Ergonomic handle
- 6: Hollow tip
- 7: Light source
- 8: Miniature camera
- 9: Arms
- 10: Supporting member
- 11: LCD screen
- 12: Single outlet port

## Claims

1. MEDICAL DEVICE FOR EXPLORING AND OPERATING ON THE ABDOMINAL CAVITY AND THE PELVIC CAVITY **characterized by** a main body (2) with two lateral working channels (2) and a central channel (3) with a source of light (7) and a miniature camera (8) to introduce surgical instruments (4), with an ergonomic handle (5) with a hollow tip (6) which allows the passing of the camera (8), with a source of light (7) and a miniature camera on its tip (11), with three arms (9) with a support (10) to place a LCD screen (11), which receives the images the camera transmits (8).

2. MEDICAL DEVICE FOR EXPLORING AND OPERATING ON THE ABDOMINAL CAVITY AND THE PELVIC CAVITY **characterized** because composed according to claim 1, by a main body (1), with two lateral working channels (2) in order to introduce surgical instruments (4) which come out to a common hole (12).

3. MEDICAL DEVICE FOR EXPLORING AND OPERATING ON THE ABDOMINAL CAVITY AND THE PELVIC CAVITY **characterized** because according to claim 1, it has a LCD screen (11) connected to the main body (1) though a support (10) which allows moving and removing the screen (11).

4. MEDICAL DEVICE FOR EXPLORING AND OPERATING ON THE ABDOMINAL CAVITY AND THE PELVIC CAVITY **characterized by** two arms (9) with an opening and closing movement, located on the extreme part of the main body (1).
